# EUROPEAN PATENT APPLICATION

(11) **EP 4 721 852 A1**
(43) Date of publication of application: **08.04.2026**
(21) Application number: 24901024.0
(22) Date of filing: 03.12.2024
(51) Int. Cl.: B01J 8/00, B01J 8/02, B01J 38/04, C07C 51/377, C07C 57/04

(54) **METHOD AND APPARATUS FOR REGENERATION OF FIXED BED CATALYTIC REACTOR**

(30) Priority: 07.12.2023 KR 20230176551; 02.12.2024 KR 20240176683
(71) Applicant: LG Chem, Ltd., Seoul 07336 (KR)
(72) Inventor: SHIN, Daeyoung, Daejeon 34122 (KR); KIM, Mi Kyung, Daejeon 34122 (KR); JEONG, Hoiin, Daejeon 34122 (KR); IM, Yong O, Daejeon 34122 (KR); JUNG, Dabin, Daejeon 34122 (KR); LEE, Jiyoung, Daejeon 34122 (KR); KANG, Tae Hun, Daejeon 34122 (KR); KIM, Hyunji, Daejeon 34122 (KR); KIM, Seungik, Daejeon 34122 (KR); LEE, Jongheon, Daejeon 34122 (KR)
(74) Representative: Goddar, Heinz J.
(86) International application number: PCT/KR2024/019591
(87) International publication number: WO 2025/121838

(57) **Abstract**

The present disclosure relates to a method and apparatus for regeneration of fixed bed catalytic reactor, and more particularly, to a method and apparatus for regeneration of a catalyst in the process of producing acrylic acid from lactic acid.

## Description

### [TECHNICAL FIELD]

### Cross-Reference to Related Application(s)

This application claims priority to and the benefit of Korean Patent Application No. 10-2023-0176551, filed on December 7, 2023, and Korean Patent Application No. 10-2024-0176683, filed on December 2, 2024, with the Korean Intellectual Property Office, the disclosures of which are incorporated herein by reference in their entirety.

The present disclosure relates to a method and apparatus for regeneration of fixed bed catalytic reactor, and more particularly, to a method and apparatus for regeneration of a catalyst in the process of producing acrylic acid from lactic acid.

### [BACKGROUND]

Acrylic acid is an organic compound having both a carboxylic acid and an unsaturated double bond in its molecule, and is used in various industrial fields because it has an extremely simple structure and can be polymerized while being converted into various materials.

Specifically, acrylic acid can be used as polyacrylic acid, tacky-adhesives, paints, etc. required for the production of a superabsorbent polymer, or may be used as a raw material for the production of other types of acrylate-based monomers, or may also be used as a raw material for polymerization with various other monomers such as acrylamide, acrylonitrile, styrene, and alpha olefins.

This acrylic acid is generally produced by using propylene produced in the refining and separation process of crude oil, such as naphtha cracking.

However, recently, as concerns about the depletion of crude oil and environmental issues are increasing, interests in the method for producing acrylic acid using environmentally friendly raw materials are increasing.

### [DETAILED DESCRIPTION OF THE INVENTION]

### [Technical Problem]

It is an object of the present disclosure to provide a method and apparatus for regeneration of fixed bed catalytic reactor that can stably regenerate a catalyst while reducing catalyst regeneration costs.

### [Technical Solution]

In order to achieve the above object, according to the present disclosure, there is provided a method for regeneration of fixed bed catalytic reactor, the method comprising the steps of: supplying a feed containing lactic acid gas to a first reactor filled with a catalyst to progress a lactic acid dehydration reaction; separating acrylic acid and by-product from the reaction product; adding the by-product into a regenerative thermal oxidizer(RTO); and adding a gas discharged from the regenerative thermal oxidizer into a second reactor filled with a catalyst to regenerate the catalyst.

According to one embodiment, the reaction product may contain 5 to 30% by weight of acrylic acid, and 1 to 15% by weight of by-product except water.

According to one embodiment, the by-product added into the regenerative thermal oxidizer may include at least one selected from the group consisting of acetaldehyde, 2,3-pentanedione, lactic acid, carbon dioxide, and carbon monoxide.

According to one embodiment, the regenerative thermal oxidizer may be operated under a temperature condition of 300 to 900°C.

According to one embodiment, the gas discharged from the regenerative thermal oxidizer may contain 0.5 to 10% by volume of oxygen.

According to one embodiment, the regeneration method may further comprise a step of controlling the temperature of the gas discharged from the regenerative thermal oxidizer to 100 to 500°C.

According to one embodiment, the second reactor may be operated at 300 to 500°C.

In addition, according to the present disclosure, there is provided an apparatus for regeneration of fixed bed catalytic reactor, the apparatus comprising: a first reactor of a fixed bed isothermal type that is filled with a catalyst; a separator that separates the product of the first reactor; a regenerative thermal oxidizer that combusts the by-product discharged from an upper part of the separator; and a second reactor of a fixed bed isothermal type that is filled with a catalyst therein and is supplied with gas discharged from the regenerative thermal oxidizer.

According to one embodiment, the regeneration apparatus may further comprise a heat exchanger that cools the gas discharged from the regenerative thermal oxidizer and supplies it to the second reactor. The heat exchanger may be a steam generator.

As used herein, the terms "a first," "a second," etc. are used herein to explain various constitutional elements, and these terms are used only to distinguish one constitutional element from another constitutional element.

Further, the technical terms used herein is only to explain exemplary embodiments and is not intended to limit the scope of the present disclosure.

The singular forms "a," "an" and "the" are intended to include plural forms, unless the context clearly indicates otherwise.

It should be understood that the terms "comprise," "include", "have", etc. are used herein to specify the presence of stated features, integers, steps, components or combinations thereof, but do not preclude the presence or addition of one or more other features, integers, steps, components, or combinations thereof.

Also, as used herein, in case a layer or an element is mentioned to be formed "on" or "above" layers or elements, it means that the layer or element is directly formed on the layers or elements, or it means that other layers or elements may be additionally formed between the layers, on a subject, or on a substrate.

Although the present disclosure may have various forms and various modifications may be made thereto, specific examples will be exemplified and explained in detail below. However, it is not intended to limit the present disclosure to specific disclosure, and it should be understood that the present disclosure includes all the modifications, equivalents or replacements thereof without departing from the spirit and technical scope of the present disclosure.

Hereinafter, the present disclosure is discussed in detail.

According to an aspect of the present disclosure, there is provided a method for regeneration of fixed bed catalytic reactor, the method comprising the steps of: supplying a feed containing lactic acid gas to a first reactor filled with a catalyst to progress a lactic acid dehydration reaction; separating acrylic acid and by-product from the reaction product; adding the by-product into a regenerative thermal oxidizer(RTO); and adding a gas discharged from the regenerative thermal oxidizer into a second reactor filled with a catalyst to regenerate the catalyst.

The process of producing acrylic acid through the gas-phase dehydration of lactic acid is mainly progressed under an acid catalyst. At this time, lactic acid as a reactant, acrylic acid as a product, and olefin compounds as an intermediate product are contained during the reaction process, and thus frequently form coke on the catalyst surface. The coke formed on the catalyst surface can block an active site of the catalyst and cause a deactivation of the catalyst. Further, the differential pressure before and after the catalyst layer increases rapidly due to the accumulated coke, which may interrupt the process. As a result, coke excessively deposited on the catalyst layer inhibits the yield of acrylic acid which is the target product.

Therefore, the catalyst used in the method for producing acrylic acid through a gas-phase dehydration reaction of lactic acid requires a process of removing coke formed on the catalyst layer through a regeneration process. Coke is a carbon deposit, and in order to remove the coke, it is common to stop the reactor operation and inject air containing oxygen to combust and remove the coke. At this time, the combustion reaction of coke is an exothermic reaction, and a hot spot is formed on the catalyst, so that a sintering phenomenon of the catalyst may appear during the progress of the regeneration at a high temperature.

Thus, in the process of producing acrylic acid through the gas-phase dehydration reaction of lactic acid, it is important to control a hot spot temperature of the catalyst in the catalyst regeneration process. It is common to use an inert gas together with oxygen to control the hot spot temperature during catalyst regeneration and lower the oxygen partial pressure. If nitrogen, which is mainly used as an inert gas, is separately added during regeneration, it is the cause of increasing the process costs.

Thus, the present inventors have found that in order to reduce the cost of the regeneration process, a gas discharged from the regenerative thermal oxidizer for treating by-product after the production of acrylic acid is used as a high-temperature inert gas, thereby capable of reducing process costs, and completed the present disclosure.

First, the method for producing a feed containing a lactic acid gas, which is a reaction product of the present disclosure, is not particularly limited, and the feed may be produced by a conventional method in the art, but the method described below can be applied as an example.

The lactic acid gas can be produced by vaporizing a lactic acid aqueous solution. The lactic acid aqueous solution may preferably have a concentration range of about 10 to about 80 wt.%.

If the concentration of lactic acid is too low, the efficiency of the vaporization step and the efficiency of the dehydration reaction of the first step may become too low, and if the concentration of lactic acid is too high, the content of oligomers and the like in the aqueous solution becomes high, which may cause a problem that the efficiency of the dehydration reaction is decreased and the production of by-product is promoted.

Further, the vaporization reaction of lactic acid can be progressed under a temperature condition of about 200°C to 300°C. If the temperature of the vaporization reaction is too low, the vaporization efficiency may be decreased, and if the temperature of the vaporization reaction is too high, the decarboxylation or decarbonylation of the vaporized lactic acid molecules may be mainly progressed, which may cause a problem that the production of aldehyde is promoted.

Then, a feed containing lactic acid gas can be supplied to the reactor filled with a catalyst to progress a lactic acid dehydration reaction.

The feed containing the lactic acid gas may be continuously supplied to a catalyst part where a catalyst is present in accordance with the flow of a carrier gas. The carrier gas may be an inert gas, and nitrogen or the like can be used.

Further, the reaction may be progressed under a temperature condition of about 300°C to about 400°C. If the reaction temperature is too low, the lactic acid conversion rate and acrylic acid yield may be decreased, and if the reaction temperature is too high, there may be a problem that the amount of formation of by-products is increased.

Further, the catalyst may include at least one selected from the group consisting of a calcium phosphate-based catalyst, a sodium phosphate-based catalyst, and an aluminum phosphate-based catalyst, and the other reaction conditions may be used without particular limitation as long as they are generally used in the technical field to which the present disclosure pertains, unless they are contrary to the contents defined herein. As an example, the catalyst may include CaSO₄/Na₂SO₄; Na₄P₂O₇/CaSO₄; Na₄P₂O₇/Ca₃(PO₄)₂; NaH₂PO₄-NaHCO₃/SiO₂; AlPO₄-NH₃; Ca₃(PO₄)₂/CaSO₄; Ca₂P₂O₇; Ca₅(PO₄)₃(OH), and the like.

Further, according to one embodiment, the production method may be a batch type or a continuous type, but it is preferably a continuous type equipped with a catalyst layer in which lactic acid gas for reaction is continuously transferred to a reactor and acrylic acid, which is a product of the dehydration reaction, is also continuously obtained.

According to one embodiment, the reaction product may contain 5 to 30% by weight of acrylic acid, and 1 to 15% by weight of by-product except water. Preferably, the reaction product may contain 8 to 28% by weight, or 10 to 25% by weight of acrylic acid, and 3 to 12% by weight, or 5 to 10% by weight of by-product except water.

During the progress of the lactic acid dehydration reaction, a by-product may be generated together due to acrylic acid and side reactions. The by-product may, in addition to acrylic acid, include water, acetaldehyde, carbon monoxide, carbon dioxide, diluted gas, a low boiling point by-product, and a high boiling point by-product, and may also include unreacted lactic acid. Thus, a step of separating the target product, i.e., acrylic acid, and by-product from the reaction product formed after the above-mentioned lactic acid dehydration reaction step.

The step of separating acrylic acid and by-product may be progressed by applying a process commonly used in the art. As an example, the reaction product stream may be supplied to a cooling tower to be condensed, and then the discharge stream containing acrylic acid can be supplied to a distillation tower to separate acrylic acid and by-product through distillation, alternatively the reaction product may be condensed in multiple cooling towers and then the acrylic acid and by-product may be separated from each other using an extractant in an extraction tower.

In addition, in the above process, devices such as a heat exchanger, a valve, a pump, a separator, and a mixer may be further included, if necessary.

The present disclosure includes a step of adding a by-product separated from the reaction product containing acrylic acid into a regenerative thermal oxidizer(RTO). Generally, the regenerative thermal oxidizer refers to a device that stores waste heat of discharge gas through direct contact, reuses the stored heat, and incinerates and removes volatile organic compound gases generated in the process. The regenerative thermal oxidizer may include a combustion chamber and a predetermined number of beds made of a heat storage agent to increase the heat recovery rate.

According to one embodiment, the by-product added into the regenerative thermal oxidizer may include at least one selected from the group consisting of acetaldehyde, 2,3-pentanedione, lactic acid, carbon dioxide, carbon monoxide, and the like.

According to one embodiment, the regenerative thermal oxidizer can be operated under a temperature condition of 300 to 900°C. If the temperature is less than 300°C, complete combustion may not be achieved, and if the temperature is more than 900°C, there may be a problem of thermal damage to the regenerative thermal oxidizer in the regenerative thermal oxidizer. Preferably, the regenerative thermal oxidizer can be operated under a temperature condition of 300 to 900°C, 400 to 850°C, or 500 to 800°C. Meanwhile, the regenerative thermal oxidizer is operated at a temperature in the above range, so that the temperature of the gas discharged from the regenerative thermal oxidizer can be adjusted to the above-mentioned range.

Further, a ccording to one embodiment, the gas discharged from the regenerative thermal oxidizer may include 0.5 to 10% by volume of oxygen. The inclusion of oxygen in the above range can provide an oxygen partial pressure suitable for catalyst regeneration in the second reactor described below, so that the hot spot of the catalyst may be adjusted to an appropriate temperature during the coke combustion process. Preferably, the gas may include oxygen at 0.5 to 10% by volume, 1 to 8% by volume, or 2 to 5% by volume.

In addition, the gas discharged from the regenerative thermal oxidizer may, in addition to oxygen, further include nitrogen, carbon dioxide, carbon monoxide, water, and the like.

According to one embodiment, a step of adjusting the temperature of the gas discharged from the regenerative thermal oxidizer to 100 to 500°C may be further included. As an example, the step may be progressed by a method which comprises adding a gas discharged from the regenerative thermal oxidizer into a steam generator to generate steam through heat exchange, and adjusting the temperature of the RTO discharge gas. The adjustment of the temperature of the gas discharged from the regenerative thermal oxidizer to the temperature range described above allows the temperature to be controlled to a temperature suitable for catalyst regeneration in the second reactor described below, and the hot spot of the catalyst to an appropriate temperature in the coke combustion process. Preferably, the gas may be discharged at 150 to 450°C, 200 to 400°C, or 250 to 350°C. Meanwhile, the steam generator is a heat exchanger that receives supply of heat from a high-temperature heat source (gas discharged from the regenerative thermal oxidizer) and boils BFW (boiling feed water) to create steam. Thereby, the gas discharged from the regenerative thermal oxidizer can be cooled to lower its temperature to an appropriate level.

Meanwhile, the gas discharged from the above-mentioned regenerative thermal oxidizer is added into the second reactor of the present disclosure. The second reactor of the present disclosure is a reactor after the progress of the lactic acid dehydration reaction in a form filled with a catalyst, which is a reactor in which coke is formed on the catalyst layer inside the reactor. In the conventional catalyst regeneration process, a gas stream containing a separate inert gas and oxygen is added into the reactor to progress the catalyst regeneration process as mentioned above, however, in the present disclosure, the by-product treatment and catalyst regeneration can be efficiently progressed by using the above-mentioned regenerative thermal oxidizer.

According to one embodiment, the second reactor may be operated at 300 to 500°C. If the temperature of the second reactor is too high, there may be a problem of shortening the catalyst life due to thermal shock, and if the temperature is too low, there may be a problem that the efficiency of coke removal decreases. Preferably, the temperature of the second reactor may be 300°C or more, 330°C or more, or 350°C or more, and 500°C or less, 480°C or less, or 460°C or less. Meanwhile, the temperature of the catalyst layer may be controlled to the same range by controlling the operating temperature of the second reactor.

Further, the catalyst regeneration step may be configured in the form of a swing reactor that converts the first reactor into a second reactor when the yield of acrylic acid decreases by 5 to 20% or 10 to 20% relative to a normal operation after the progress of the lactic acid dehydration reaction in the first reactor.

Meanwhile, according to the present disclosure, there is provided an apparatus for regeneration of fixed bed catalytic reactor, the apparatus comprising: a first reactor 100 of a fixed bed isothermal type that is filled with a catalyst; a separator that separates the product of the first reactor; a regenerative thermal oxidizer that combusts byproducts discharged from the upper part of the separator; and a second reactor of a fixed bed isothermal type that is filled with a catalyst therein and is supplied with gas discharged from the regenerative thermal oxidizer.

The details of the first reactor, the separator, the regenerative thermal oxidizer, and the second reactor are as described above. In addition, the apparatus may further include a heat exchanger that cools the gas discharged from the regenerative thermal oxidizer and supplies it to the second reactor, if necessary.

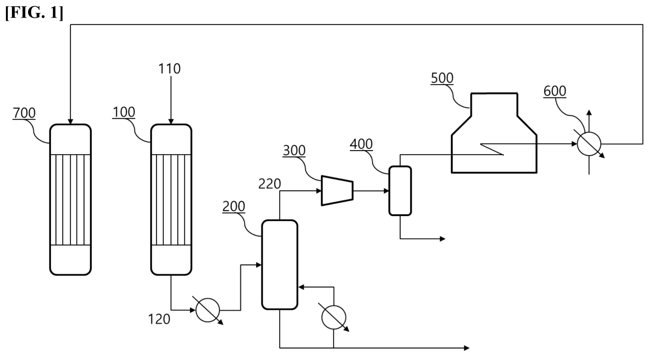

FIG. 1 is a process diagram that schematically shows a method according to an aspect of the present disclosure.

Referring to FIG. 1, a method for regeneration of fixed bed catalytic reactor according to an aspect of the present disclosure can be confirmed by a series of steps comprising: a step of supplying a feed 110 containing lactic acid gas to a first reactor 100 filled with a catalyst to progress a lactic acid dehydration reaction; a step of separating acrylic acid and by-product 220 from the reaction product 120; a step of adding the by-product into a regenerative thermal oxidizer(RTO) 500; and a step of adding a gas discharged from the regenerative thermal oxidizer into a second reactor 700 filled with a catalyst to regenerate the catalyst.

In addition, referring to FIG. 1, an apparatus for regeneration of fixed bed catalytic reactor according to an aspect of the present disclosure can be confirmed, comprising: a first reactor 100 of a fixed bed isothermal type that is filled with a catalyst; a separator 200 that separates the product of the first reactor; a regenerative thermal oxidizer 500 that combusts the by-product discharged from an upper part of the separator; and a second reactor 700 of a fixed bed isothermal type that is filled with a catalyst therein and supplied with gas discharged from the regenerative thermal oxidizer.

In addition, the apparatus for producing acrylic acid of the present disclosure may further include a compressor 300 and a gas-liquid separator 400, if necessary. In addition, the apparatus for producing acrylic acid of the present disclosure may further include a heat exchanger 600 that cools a gas discharged from the regenerative thermal oxidizer 500 and supplies it to the second reactor 700, wherein the heat exchanger may be a steam generator.

### [Advantageous Effects]

As mentioned above, according to a method and apparatus for regeneration of fixed bed catalytic reactor of the present disclosure, the catalyst can be regenerated by using a gas discharged from the regenerative thermal oxidizer, thereby reducing process costs, minimizing the thermal shock of the catalyst, and enabling stable regeneration.

### [BRIEF DESCRIPTION OF THE DRAWINGS]

FIG. 1 is a process diagram that schematically shows a method according to an aspect of the present disclosure.
FIG. 2 is a process diagram that schematically shows a catalyst regeneration apparatus according to a comparative example.

### [DETAILED DESCRIPTION OF THE EMBODIMENTS]

Hereinafter, preferred Examples are provided for better understanding of the invention. However, these Examples are for illustrative purposes only, and the invention is not intended to be limited by these Examples.

### Example 1

As shown in FIG. 1 below, a 40 wt.% lactic acid aqueous solution was vaporized at 350°C and added into a first reactor, and subjected to a dehydration reaction to prepare a reaction product containing acrylic acid (acrylic acid 17.0 wt.%, byproduct: acetaldehyde, 2,3-pentanedione, lactic acid, carbon dioxide, and carbon monoxide, etc., 10 wt.% in total).

Then, the reaction product was supplied to a separator to separate low boiling point substances and high boiling point substances in the reaction product. A cooling tower was used as the separator. In the cooling tower, the low boiling by-product (acetaldehyde, 2,3-pentanedione, lactic acid, carbon dioxide, and carbon monoxide) contained in the reaction product were discharged to an upper part of the cooling tower together with the nitrogen contained in the reaction product, and acrylic acid was discharged to a lower part together with the high boiling substance. The stream discharged to the upper part of the cooling tower was compressed through a compressor, and the condensed substance and the gas phase substance were separated in the gas-liquid separator.

Then, the gas phase substance (acetaldehyde, 2,3-pentanedione, lactic acid, carbon dioxide, and carbon monoxide) discharged from the gas-liquid separator were added into a regenerative thermal oxidizer(RTO) together with air. The regenerative thermal oxidizer was operated at a temperature of about 800 °C, and gases containing nitrogen, carbon dioxide, water, and oxygen were discharged through complete combustion. The discharge gas at about 800°C was cooled to 350°C after generating steam in a heat exchanger (steam generator) (oxygen concentration 3.5 vol.%). Then, the cooled regenerative thermal oxidizer discharge stream was added into a second reactor for catalyst regeneration where coke generated during the reaction was accumulated on the catalyst layer (catalyst at the point where the yield of acrylic acid decreased by 10%). The second reactor was allowed to regenerate the catalyst for 24 hours while maintaining the temperature at 400°C.

### Example 2

The dehydration reaction was progressed in the same manner as Example 1, and passed through a cooling tower and a gas-liquid separator to obtain a gas-phase substance. The gas-phase substance discharged from the gas-liquid separator was added into a regenerative thermal oxidizer(RTO) together with air. The regenerative thermal oxidizer was operated at a temperature of about 700°C, and gases containing nitrogen, carbon dioxide, water, and oxygen were discharged through complete combustion. The discharge gas at about 700°C was used to generate steam in a heat exchanger (steam generator), and then cooled to 390°C (oxygen concentration: 0.5 vol.%). Then, the cooled regenerative thermal oxidizer discharge stream was added into a second reactor for catalyst regeneration where coke generated during the reaction was accumulated on the catalyst layer (catalyst at the point where the yield of acrylic acid decreased by 10%). The second reactor was allowed to regenerate the catalyst for 24 hours while maintaining the temperature at 400°C.

### Example 3

A dehydration reaction was progressed in the same manner as in Example 1, and passed through a cooling tower and a gas-liquid separator to obtain a gas-phase substance. The gas-phase substance discharged from the gas-liquid separator was added into a regenerative thermal oxidizer(RTO) together with air. The regenerative thermal oxidizer was operated at a temperature of about 600°C, and gases containing nitrogen, carbon dioxide, water, and oxygen were discharged through complete combustion. The discharge gas at about 600°C was cooled to 400°C (oxygen concentration: 0.5 vol.%) after generating steam in a heat exchanger (steam generator). Then, in order to increase the oxygen concentration of the cooled regenerative thermal oxidizer discharge stream, air at 25°C was added into a stream, and a regeneration stream was prepared with an oxygen concentration of 2.0 vol% at 370°C.

The regeneration stream was added into the second reactor for catalyst regeneration of the second reactor where coke generated during the reaction was accumulated on the catalyst layer (catalyst at the point where the yield of acrylic acid decreased by 10%). The second reactor was allowed to regenerate the catalyst for 24 hours while maintaining the temperature at 400°C.

### Comparative Example

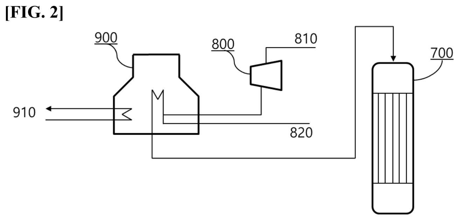

A second reactor was prepared in the same state as in Examples. As shown in FIG. 2 below, a separate regeneration gas stream was prepared for catalyst regeneration. Specifically, air and nitrogen were mixed in a volume ratio of 1:5 to prepare a regeneration gas having an oxygen concentration of 3.5 vol.%. The prepared regeneration gas was heated to 350°C in a fired heater and then added into the second reactor. The second reactor was allowed to regenerate the catalyst for 24 hours while maintaining the temperature at 400°C.

The production method of Comparative Example in which a separate regeneration stream was prepared and added may cause a problem that the catalyst regeneration cost increases due to nitrogen consumed to produce a regeneration gas having an appropriate oxygen concentration (3.5 vol%) and temperature (350°C) and LNG fuel used in the fired heater. On the other hand, the method for producing acrylic acid according to the present disclosure described above progresses a catalyst regeneration by treating waste gas and/or waste oil generated as by-products after producing acrylic acid from lactic acid through a regenerative combustion oxidizer and then using the discharged gas stream, thereby providing excellent process economic performance and enabling stable regeneration of the catalyst.

**[Description of Reference Numerals]**

| | | | |
|---|---|---|---|
| 100: | first reactor | 110: | feed |
| 120: | reaction product | 200: | separator |
| 220: | by-product | 300: | compressor |
| 400: | gas-liquid separator | 500: | regenerative thermal oxidizer(RTO) |
| 600: | heat exchanger | 700: | second reactor |
| 800: | compressor | 810: | air stream |
| 820: | nitrogen stream | 900: | fired heater |
| 910: | fuel supply unit | | |

## Claims

1. A method for regeneration of fixed bed catalytic reactor, the method comprising the steps of:
supplying a feed containing lactic acid gas to a first reactor filled with a catalyst to progress a lactic acid dehydration reaction;
separating acrylic acid and by-product from the reaction product;
adding the by-product into a regenerative thermal oxidizer(RTO); and
adding a gas discharged from the regenerative thermal oxidizer into a second reactor filled with a catalyst to regenerate the catalyst.

2. The method for regeneration of fixed bed catalytic reactor according to claim 1,
wherein the reaction product contains 5 to 30% by weight of acrylic acid, and 1 to 15% by weight of by-product except water.

3. The method for regeneration of fixed bed catalytic reactor according to claim 1,
wherein the by-product added into the regenerative thermal oxidizer includes at least one selected from the group consisting of acetaldehyde, 2,3-pentanedione, lactic acid, carbon dioxide, and carbon monoxide.

4. The method for regeneration of fixed bed catalytic reactor according to claim 1,
wherein the regenerative thermal oxidizer is operated under a temperature condition of 300 to 900°C.

5. The method for regeneration of fixed bed catalytic reactor according to claim 1,
wherein the gas discharged from the regenerative thermal oxidizer contains 0.5 to 10% by volume of oxygen.

6. The method for regeneration of fixed bed catalytic reactor according to claim 1,
further comprising controlling the temperature of the gas discharged from the regenerative thermal oxidizer to 100 to 500°C.

7. The method for regeneration of fixed bed catalytic reactor according to claim 1,
wherein the second reactor is operated at 300 to 500°C.

8. An apparatus for regeneration of fixed bed catalytic reactor, the apparatus comprising:
a first reactor of a fixed bed isothermal type that is filled with a catalyst;
a separator that separates the product of the first reactor;
a regenerative thermal oxidizer that combusts the by-product discharged from an upper part of the separator; and
a second reactor of a fixed bed isothermal type that is filled with a catalyst therein and is supplied with gas discharged from the regenerative thermal oxidizer.

9. The apparatus for regeneration of fixed bed catalytic reactor according to claim 8,
further comprising a heat exchanger that cools the gas discharged from the regenerative thermal oxidizer and supplies it to the second reactor.

10. The apparatus for regeneration of fixed bed catalytic reactor according to claim 9,
wherein the heat exchanger is a steam generator.
